# EUROPÄISCHE PATENTANMELDUNG

(11) **EP 0 955 006 A1**
(43) Veröffentlichungstag der Anmeldung: **10.11.1999**
(21) Anmeldenummer: 99115752.0
(22) Anmeldetag: 15.12.1993
(51) Int. Cl.: A61B 1/00, A61M 25/01

(54) **Vorrichtung zum Bewegen eines Endoskops in einem kanalartigen Hohlraum**

(30) Priorität: 15.12.1992 DE 4242291
(62) Teilanmeldung aus: 94903800.4
(71) Anmelder: STM Medizintechnik Starnberg GmbH, 82319 Starnberg (DE)
(72) Erfinder: Bob, Alexander, 68163 Mannheim (DE); Bob, Konstantin, 69469 Weinheim (DE)
(74) Vertreter: Tiedtke, Harro, Dipl.-Ing.

(57) **Zusammenfassung**

Vorrichtung zum Bewegen eines Endoskops (2) längs eines kanalartigen Hohlraums (4), gekennzeichnet durch: (a) einen Stülpschlauch (20), der beim Einsatz der Vorrichtung einen relativ zu der Hohlraumwand ruhenden, äußeren Bereich (26) und einen inneren Bereich (24) aufweist, der eine Teillänge des Endoskops (2) aufnimmt; (b) eine Antriebseinrichtung (18), mit der sich in dem Hohlraum (4) befindliche, vordere Teile des Endoskops (2) und des inneren Bereichs (24) des Stülpschlauchs (20) kraftbetätigt vorwärtsbewegen lassen; (c) und eine auf das Endoskop (2) wirkende Bewegungsbestimmungseinrichtung, mit der sich die Vorwärts-Bewegungsgeschwindigkeit des vorderen Teils des Endoskops (2) derart bestimmen läßt, daß sie beim Betrieb der Antriebseinrichtung (18) im wesentlichen halb so groß ist wie die Geschwindigkeit des vorderen Teils des inneren Bereichs (24) des Stülpschlauchs (20).

## Beschreibung

Die Erfindung bezieht sich auf ein Endoskop.

Endoskope sind zu einem wichtigen Hilfsmittel in der Technik und in der Medizin geworden, um kanalartige Hohlräume zu inspizieren, die auf andere Weise nicht oder nur mit erheblichen Eingriffen zugänglich sind. Endoskope sind an ihrem Distalende mit einer Beleuchtungseinrichtung und mit einer Optik zur visuellen Erfassung des davorliegenden Bereichs des Hohlraums ausgerüstet. Die am Distalende des Endoskops erfaßte, optische Information wird normalerweise entweder mittels einer Faseroptik durch das Endoskop nach hinten zu seinem Bedienungsende übertragen oder wird mittels eines Kamerachips am Distalende erfaßt, durch eine elektrische Leitung durch das Endoskop nach hinten geleitet und auf einem Bildschirmmonitor sichtbar gemacht. Insgesamt haben Endoskope, abgesehen von dem hinteren Bedienungsende, üblicherweise eine langgestreckte, biegsam-stabförmige Gestalt.

Um einen kanalartigen Hohlraum inspizieren zu können, wird das Endoskop durch eine Zugangsöffnung in den Hohlraum eingebracht. Das weitere Hineinbewegen des Endoskops geschieht üblicherweise dadurch, daß eine Bedienungsperson an dem aus der Zugangsöffnung herausragenden Teil des Endoskops mit der Hand angreift und von dort her das drucksteife Endoskop allmählich immer weiter in den Hohlraum hineinschiebt.

Dieses zunehmende Hineinbewegen des Endoskops in den kanalartigen Hohlraum ist relativ mühsam. Besonders schwierig gestaltet sich das Vorschieben des Endoskops, wenn der zu inspizierende, kanalartige Hohlraum engere Biegungen, Engstellen oder dergleichen aufweist. Wenn der kanalartige Hohlraum eine nichtglatte Wand aus einem wenig festen Material aufweist, besteht die Gefahr, daß das Distalende beim Vorschieben an der Hohlraumwand hängen bleibt; dies kann zu Beschädigungen der Hohlraumwand führen. Insbesondere wenn der Hohlraum mehrere Biegungen aufweist, wird durch das Anschieben vom hinteren Ende des Endoskops her erheblicher Druck von innen her gegen die jeweils biegungsäußeren Wandbereiche ausgeübt. Das weitere Vorschieben des Endoskops ist erschwert.

Der Erfindung liegt die Aufgabe zugrunde, einen Weg für ein komplikationsloseres Bewegen des Endoskops längs des Hohlraums mit verringerter Gefahr einer Beschädigung der Hohlraumwand verfügbar zu machen.

Zur Lösung dieser Aufgabe ist das Verfahren erfindungsgemäß dadurch gekennzeichnet,
(a) daß ein Stülpschlauch eingesetzt wird, dessen äuBerer Bereich relativ zu der Hohlraumwand ruht beim Bewegen des Endoskops längs des Hohlraums und dessen innerer Bereich eine Teillänge des Endoskops aufnimmt;
(b) und daß in dem Hohlraum befindliche, vordere Teile des Endoskops und des inneren Bereichs des Stülpschlauchs mindestens während eines Teils der Zeit derart vorwärtsbewegt werden, daß die Bewegungsgeschwindigkeit des vorderen Teils des Endoskops im wesentlichen halb so groß ist wie die Bewegungsgeschwindigkeit des vorderen Teils des inneren Bereichs des Stülpschlauchs.

Aufgrund des erfindungsgemäßen Verfahrens wird nicht nur das Endoskop in den kanalartigen Hohlraum eingebracht und längs diesem vorgeschoben, sondern es wird auch der Stülpschlauch zwischen der Hohlraumwand und dem Endoskop eingebracht. Der Stülpschlauch ergibt eine Auskleidung des Hohlraums, welche das Bewegen des Endoskops längs des Hohlraums erleichtert und die Gefahr von Beschädigungen der Hohlraumwand entscheidend verringert.

Ein gemeinsames Vorwärtsbewegen des vorderen Teils des Endoskops und des vorderen Teils des inneren Stülpschlauchbereichs führt normalerweise dazu, daß sich diese beiden vorderen Teile mit praktisch gleicher Geschwindigkeit vorwärtsbewegen. Da von einer bestimmten, vorgeschobenen Länge des inneren Stülpschlauchbereichs die Hälfte nach Durchwandern des Umstülpbereichs für den äußeren Stülpschlauchbereich "verbraucht" wird, ergäbe sich im Ergebnis, daß der vordere Teil des Endoskops um diese halbe Länge nach vorn über den Umstülpbereich des Stülpschlauchs vorsteht. Um dies zu vermeiden, wird erfindungsgemäß der vordere Teil des Endoskops mit einer Geschwindigkeit vorwärtsbewegt, die im wesentlichen halb so groß wie die Bewegungsgeschwindigkeit des vorderen Teils des inneren Stülpschlauchbereichs ist. Hiermit einher geht eine Relativ-Gleitbewegung des inneren Stülpschlauchbereichs relativ zu dem Endoskop.

Eine bevorzugte, konkrete Möglichkeit zur Erreichung des genannten Geschwindigkeitsverhältnisses besteht darin, Vorwärts-Antriebskraft auf den vorderen Teil des Endoskops und den vorderen Teil des inneren Bereichs des Stülpschlauchs auszuüben und gleichzeitig Rückwärts-Bewegungsbestimmungskraft auf einen hinten aus dem Stülpschlauch herausreichenden, hinteren Teil des Endoskops auszuüben.

Der erfindungsgemäße Bewegungsablauf der vorderen Teile des Endoskops und des inneren Stülpschlauchbereichs kann grundsätzlich für die gesamte Vorwärtsbewegungsstrecke kontinuierlich durchgeführt werden. Man kann aber auch die Gesamtbewegungsstrecke auf mehrere, nacheinander mit Zeitabstand erfolgende Bewegungsschritte aufteilen.

Die Erfindung läßt die Möglichkeit, das Endoskop zu Zeiten, in denen keine Vorwärtsbewegung der vorderen Teile von Endoskop und Stülpschlauch mit dem beschriebenen Geschwindigkeitsverhältnis stattfindet, relativ zu dem inneren Bereich des Stülpschlauchs zu verschieben, also einfach in der geschaffenen Auskleidung längs des Hohlraums zu bewegen. Eine typische Situation ist zum Beispiel, daß die Bedienungsperson sieht, daß sich vor dem Distalende des Endoskops ein ziemlich gerader Hohlraumabschnitt mit einigermaßen glatter Wand befindet. Dann kann die Bedienungsperson das Endoskop relativ zu dem als Ganzes ruhenden Stülpschlauch ein ganzes Stück vorschieben und dabei diesen Hohlraumabschnitt inspizieren. Ein weiteres Beispiel ist das teilweise oder gänzliche Herausziehen des Endoskops bei insgesamt ruhendem Stülpschlauch; der Stülpschlauch kann dann anschließend durch Ziehen an seinem inneren Bereich aus dem Hohlraum herausbewegt werden.

In bevorzugter Weiterbildung der Erfindung kann man die Möglichkeit vorsehen, den Raum zwischen dem äußeren Bereich und dem inneren Bereich des Stülpschlauchs mittels eines Fluids mit Druck zu beaufschlagen. Als Fluid eignen sich Flüssigkeiten oder Gase. Infolge dieser Druckbeaufschlagung werden der äußere Bereich und der innere Bereich des Stülpschlauchs auseinanderbewegt oder auseinandergehalten, mit der Folge, daß die Reibung zwischen diesen beiden Bereichen minimiert wird. Der innere Bereich des Stülpschlauchs wird in Längsrichtung straff gehalten. Außerdem ergibt sich ein Vortriebseffekt auf den inneren Bereich des Stülpschlauchs, weil der in dem genannten Raum herrschende Überdruck den Umstülpbereich des Stülpschlauchs nach vorn zu bewegen trachtet. Schließlich kann sich durch diesen Überdruck ein Anpressen des inneren Bereichs des Stülpschlauchs gegen das Endoskop auf großer Länge ergeben, was einen mitziehenden Vortriebseffekt auf das Endoskop ergibt. Es versteht sich, daß der Raum zwischen dem äußeren Bereich und dem inneren Bereich des Stülpschlauchs an einer von dem Umstülpbereich beabstandeten Stelle abgeschlossen sein muß, um die beschriebene Druckbeaufschlagung zustandebringen zu können.

Ferner ist es möglich, das Fluid aus dem Raum zwischen dem äußeren Bereich und dem inneren Bereich des Stülpschlauchs abströmen zu lassen oder abzusaugen, woraufhin sich dieser Raum unter der Wirkung des äußeren Luftdrucks abflacht, also der innere Bereich des Stülpschlauchs von dem Endoskop freikommt. Dies ist günstig insbesondere für Bewegungen des Endoskops relativ zu dem insgesamt ruhenden Stülpschlauch.

Vorzugsweise befindet sich in dem Raum zwischen dem Endoskop und dem inneren Bereich des Stülpschlauchs ein Schmiermittel, wodurch die Reibung zwischen diesen beiden Elementen bei ihren Relativbewegungen verringert wird. Man kann weiteres Schmiermittel kontinuierlich oder wiederholt in diesem Raum einpressen.

Die Erfindung bezieht sich ferner auf eine Vorrichtung zum Bewegen eines Endoskops längs eines kanalartigen Hohlraums, gekennzeichnet durch:
(a) einen Stülpschlauch, der beim Einsatz der Vorrichtung einen relativ zu der Hohlraumwand ruhenden, äußeren Bereich und einen inneren Bereich aufweist, der eine Teillänge des Endoskops aufnimmt;
(b) eine Antriebseinrichtung, mit der sich in dem Hohlraum befindliche, vordere Teile des Endoskops und des inneren Bereichs des Stülpschlauchs kraftbetätigt vorwärtsbewegen lassen;
(c) und eine auf das Endoskop wirkende Bewegungsbestimmungseinrichtung, mit der sich die Vorwärts-Bewegungsgeschwindigkeit des vorderen Teils des Endoskops derart bestimmen läßt, daß sie beim Betrieb der Antriebseinrichtung im wesentlichen halb so groß ist wie die Geschwindigkeit des vorderen Teils des inneren Bereichs des Stülpschlauchs.

Aufgrund dieser erfindungsgemäßen Ausbildung der Vorrichtung ergeben sich Effekte und Vorteile, wie sie weiter vorn im Zusammenhang mit dem erfindungsgemäßen Verfahren beschrieben worden sind.

Man kann zu Beginn des Hineinbewegens des Endoskops und des Stülpschlauchs in den Hohlraum und während des weiteren Verlaufs des Hineinbewegens eine Vorratslänge von Stülpschlauch und Endoskop in geradliniger Lage vorsehen. Insbesondere bei einer beträchtlichen einzuführenden Länge des Endoskops ist es jedoch aus Platzgründen häufig bevorzugt, daß ein Teil des Stülpschlauchs und - darin aufgenommen - ein Teil des Endoskops in nicht-geradliniger Lage als beim Vorwärtsbewegen des vorderen Teils des Endoskops längs des Hohlraums zu verringernder Vorrat vorgesehen ist, wobei ein hinterer Teil des Endoskops hinten aus dem Vorratsteil des Stülpschlauchs herausreicht; und daß die Bewegungsbestimmungseinrichtung auf das Endoskop in dessen hinteren Teil wirkt. Es kann ein Vorratsstraffungselement vorgesehen sein, das eine Gegenkraft gegen eine Begradigung des Vorrats ausübt;

Es sei an dieser Stelle betont, daß - je nach Aufbau der Vorrichtung - die Bewegungsbestimmungseinrichtung insbesondere eher die Funktion hat, die Vorwärts-Bewegungsgeschwindigkeit des Endoskops hemmend auf die gewünschte Geschwindigkeit herabzusetzen, oder insbesondere eher die Funktion hat, das Endoskop in Rückwärtsrichtung relativ zu dem hinteren Ende des Stülpschlauchs anzutreiben. Die Bewegungsbestimmungseinrichtung soll vorzugsweise dem Endoskop eine vorbestimmte, definierte Geschwindigkeit mitteilen, damit das weiter vorn beschriebene Geschwindigkeitsverhältnis zwischen dem Endoskop und dem inneren Stülpschlauchbereich erreicht wird.

Vorzugsweise weist die Antriebseinrichtung eine Anpreßeinrichtung zur Schaffung eines reibschlüssigen Eingriffs mit einem Längenabschnitt des inneren Bereichs des Stülpschlauchs auf. Normalerweise geht hiermit einher, daß der Längenabschnitt gegen das Endoskop gedrückt wird, so daß auch dort Vorwärts-Bewegungskraft übertragen wird. Eine technisch besonders elegante Ausführung der Anpreßeinrichtung besteht darin, ein in einer Hülse angeordnetes, den Längenabschnitt des inneren Bereichs des Stülpschlauchs umgebendes Anpreß-Schlauchstück vorzusehen und den geschlossenen Raum zwischen der Hülse und dem Anpreß-Schlauchstück mit einem Fluid mit Druck zu beaufschlagen. Die innere Oberfläche des Anpreß-Schlauchstücks kann eine dem reibschlüssigen Eingriff mit dem Stülpschlauch förderliche Ausbildung haben, z.B. eine gezielt nicht-glatte Ausbildung haben, mit einem Reibbelag versehen sein, aus einem Material mit höherem Reibungskoeffizienten bestehen etc.. Die so ausgebildete Anpreßeinrichtung liefert zugleich, wenn sie betätigt ist, eine Abdichtung des Raums zwischen dem inneren Bereich und dem äußeren Bereich des Stülpschlauchs gegen die Umgebung an dieser Stelle.

In bevorzugter Ausgestaltung der Erfindung weist die Antriebseinrichtung einen auf den Anpreß-Längenabschnitt von Stülpschlauch und Endoskop - direkt oder indirekt - arbeitenden Elektromotor auf.

Eine als besonders einfach bevorzugte Art der Ausbildung der Bewegungsbestimmungseinrichtung besteht darin, ein Paar von elektromotorisch antreibbaren, auf das Endoskop arbeitenden Antriebsrädern vorzusehen.

Ferner kann man eine Antriebseinrichtung vorsehen, mit der sich das Endoskop relativ zu dem ruhenden, inneren Bereich des Stülpschlauchs bewegen läßt. Diese Antriebseinrichtung kann insbesondere ebenfalls von dem Paar von elektromotorisch antreibbaren, auf das Endoskop arbeitenden Antriebsrädern gebildet sein. Zu diesem Zweck können diese Antriebsräder wahlweise in beiden Drehrichtungen antreibbar sein.

Man kann eine Druckfluid-Zuführeinrichtung vorsehen, mit der sich Druckfluid dem Raum zwischen dem äußeren Bereich und dem inneren Bereich des Stülpschlauchs zuführen läßt. Ferner ist vorzugsweise eine Schmiermittel-Einpreßeinrichtung, mit der sich Schmiermittel in den Raum zwischen dem Endoskop und dem inneren Bereich des Stülpschlauchs einpressen läßt, vorgesehen. Die mit einer Druckfluid-Zuführeinrichtung und mit einer Schmiermittel-Einpreßeinrichtung einhergehenden Vorteile sind weiter vorn bereits abgehandelt worden.

Es gibt die Möglichkeit, den Stülpschlauch nicht nur vorne zur Ausbildung eines äußeren Bereichs und eines inneren Bereichs umzustülpen, sondern dies zusätzlich auch im hinteren Bereich, der sich beim Betrieb der Vorrichtung außerhalb des kanalartigen Hohlraums befindet, vorzusehen. Beim Hineinbewegen des Endoskops in den Hohraum wird dann am vorderen Ende der äußere, ruhende Bereich des Stülpschlauchs immer länger und wird am hinteren Ende der äußere, ruhende Bereich des Stülpschlauchs immer kürzer. Diese Ausbildung der erfindungsgemäßen Vorrichtung bietet Vorteile hinsichtlich der erforderlichen Länge des Endoskops, wie weiter unten bei der Beschreibung von Ausführungsbeispielen noch deutlicher werden wird.

Zur Reibungsverminderung kann der Stülpschlauch außenseitig (und damit auch innenseitig im umgestülpten, äußeren Bereich) und/oder innenseitig mit einem reibungsarmen Material, z. B. Polytetrafluorethylen, beschichtet sein.

Zum Rückwärts-Bewegen des Endoskops besteht eine erste Möglichkeit darin, einfach das Endoskop in dem ruhenden, inneren Bereich des Stülpschlauchs zurückzuziehen und danach den Stülpschlauch aus dem Hohlraum durch Ziehen an seinem inneren Bereich herauszuholen. Eine zweite Möglichkeit besteht darin, umgekehrt zu verfahren wie beim Vorwärts-Bewegen, d.h. das Endoskop mit im wesentlichen der halben Geschwindigkeit des inneren Bereichs des Stülpschlauchs rückwärts zu bewegen. Die Bewegungsbestimmungseinrichtung übt dann - in umgekehrter Richtung wie beim Vorwärts-Bewegen - eine Kraft auf das Endoskop aus, die das beschriebene Geschwindigkeitsverhältnis sicherstellt. Diese Art der Rückwärts-Bewegung hat den Vorteil, daß der kanalartige Hohlraum ein zweites Mal inspiziert werden kann.

Weiterer Gegenstand der Erfindung ist ein Verfahren zum Bewegen eines Endoskops längs eines kanalartigen Hohlraums, dadurch gekennzeichnet,
a) daß ein Stülpschlauch eingesetzt wird, dessen äußerer Bereich relativ zu der Hohlraumwand ruht beim Bewegen des Endoskops längs des Hohlraums und dessen innerer Bereich eine Teillänge des Endoskops aufnimmt;
b) daß das Bewegen des Endoskops in einer Abfolge von Bewegungsschritten in einer ersten Richtung und einer entgegengesetzten zweiten Richtung erfolgt,
c) wobei bei den Bewegungsschritten in der ersten Richtung das Endoskop gemeinsam mit dem inneren Bereich des Stülpschlauchs bewegt wird
d) und bei den Bewegungsschritten in der zweiten Richtung das Endoskop relativ zu dem inneren Bereich des Stülpschlauchs bewegt wird;
e) wobei die Bewegungsschritte in der ersten Richtung in der Summe eine größere Länge ergeben als die Bewegungsschritte in der zweiten Richtung.

Bei dieser Variante der Erfindung bewegt sich das Endoskop bei den Bewegungsschritten in der ersten Richtung gemeinsam mit dem inneren Bereich des Stülpschlauchs. Die Bewegungsschritte in der entgegengesetzten, zweiten Richtung vollführt das Endoskop ohne Mitbewegen des Stülpschlauchs, wodurch sich erreichen läßt, daß der Umstülpbereich des Stülpschlauchs nicht gegenüber dem Endoskop zurückbleibt.

Am sinnvolsten ist es, die Bewegungsschritte in der zweiten Richtung halb so groß wie die Bewegungsschritte in der ersten Richtung zu machen, wodurch im Ergebnis das Endoskop und der Stülpschlauch gemeinsam immer weiter in den Hohlraum hinein bewegt werden. Wenn die Bewegungsschritte des Endoskops in der zweiten Richtung kleiner sind als die Hälfte der Bewegungsschritte in der ersten Richtung, kommt im Lauf der Zeit das Distalende des Endoskops immer weiter nach vorn über den Umstülpbereich des Stülpschlauchs hinaus. Wenn die Bewegungsschritte des Endoskops in der zweiten Richtung größer sind als die Hälfte der Bewegungsschritte in der ersten Richtung, bleibt das Distalende des Endoskops zunehmend gegenüber dem Umstülpbereich des Stülpschlauchs zurück.

Am praktischsten ist es, die Bewegungsschritte in der ersten Richtung abwechselnd mit den Bewegungsschritten in der zweiten Richtung durchzuführen. Es ist aber auch möglich - um ein Beispiel unter vielen zu nennen - zwei aufeinanderfolgende Bewegungsschritte in der ersten Richtung durchzuführen, dann einen Bewegungsschritt in der zweiten Richtung, usw.

Beim Bewegen des Endoskops immer weiter in den Hohlraum hinein ist die erste Richtung die Hineinbewegungsrichtung und ist die zweite Richtung die Herausbewegungsrichtung. Beim Bewegen des Endoskops rückwärts immer weiter aus dem Hohlraum heraus ist die erste Richtung die Herausbewegungsrichtung und ist die zweite Richtung die Hineinbewegungsrichtung. Es wird betont, daß das erfindungsgemäße Verfahren zum Bewegen des Endoskops bei dessen Hineinbewegen in den Hohlraum und/oder bei dessen Herausbewegen aus dem Hohlraum eingesetzt werden kann.

Das erfindungsgemäße Verfahren eignet sich besonders gut dafür, die geschilderten Bewegungsschritte nicht durch manuelle Einwirkung auf das Endoskop und den Stülpschlauch durchzuführen, sondern hierfür Fremdkraft einzusetzen. Hierfür gibt es eine ganze Reihe technischer Möglichkeiten, z.B. Elektromotoren, Zylinder-Kolben-Einheiten und dergleichen, wobei das weiter unten beschriebene Ausführungsbeispiel eine konkrete Möglichkeit detailliert wiedergibt. Das Bewegen des Endoskops längs des kanalartigen Hohlraums kann quasi-automatisiert, aber kontrolliert von der Bedienungsperson, erfolgen.

Das Bewegen des Endoskops und des inneren Bereichs des Stülpschlauchs in der ersten Richtung gestaltet sich besonders einfach, wenn diese beiden Elemente als Einheit bewegt werden. Zu diesem Zweck kann man den inneren Bereich des Stülpschlauchs und das Endoskop für die Bewegungsschritte in der ersten Richtung miteinander verkoppeln.

Weiterer Gegenstand der Erfindung ist eine Vorrichtung zum Bewegen eines Endoskops längs eines kanalartigen Hohlraums, gekennzeichnet durch:
a) einen Stülpschlauch, der bei Einsatz der Vorrichtung einen relativ zu der Hohlraum ruhenden, äußeren Bereich und einen inneren Bereich aufweist, der eine Teillänge des Endoskops aufnimmt;
b) eine erste Antriebseinrichtung, mit der sich das Endoskop und der innere Bereich des Stülpschlauchs mit im wesentlichen gleicher Geschwindigkeit kraftbetätigt und schrittweise in einer ersten Richtung bewegen lassen; und
c) eine zweite Antriebseinrichtung, mit der sich das Endoskop kraftbetätigt und schrittweise in einer zweiten Richtung bewegen läßt, die der ersten Richtung entgegengesetzt ist.

Die Erfindung läßt sich zum einen bei der Inspektion, aber auch bei der Durchführung von Arbeiten, in technischen Anlagen und Einrichtungen einsetzen. Als Beispiele unter vielen seien Kernreaktoren, chemische Anlagen, Rohrleitungssysteme, genannt. Zum anderen läßt sich die Erfindung günstig auf dem Gebiet der Medizin einsetzen, insbesondere zur Exploration von Hohlräumen oder röhrenartigen Kanälen des Körpers. Endoskope haben sich besonders eingeführt zur Exploration der Speiseröhre, des Magens, des Zwölffingerdarms vom Magen aus, des Darms vom Anus aus, der Harnröhre, der Blase und der Harnleiter. Mit Hilfe von Endoskopen lassen sich heute minimal invasive Operationstechniken durchführen. Endoskope weisen in der Regel einen sogenannten Arbeitskanal auf, durch den diverse Arbeitselemente eingeführt und bedient werden können, z.B. kleine Zangen zur Entnahme von Gewebsproben, Biopsienadeln, beheizbare Schneiddrähte, kleine Scheren, Koagulationselektroden oder dergleichen. Schließlich sind in der Regel ein Fluidkanal für Spülflüssigkeit und Bedienungsdrähte zum Abwinkeln des Distalendes des Endoskops in mehrere Richtungen vorhanden. Insgesamt soll der Begriff "Endoskop" der vorliegenden Anmeldung alle Arten von längs eines kanalartigen Hohlraums zu bewegenden Instrumenten oder Einrichtungen umfassen, auch wenn der Gesichtspunkt des optischen Inspizierens nicht im Vordergrund steht oder überhaupt kein optisches Inspizieren stattfindet.

Ferner gibt die Erfindung die Möglichkeit, zunächst mit einem Endoskop unter Sichtkontrolle eine bestimmte Stelle eines kanalartigen Hohlraums anzufahren, dann das Endoskop aus dem auskleidenden Stülpschlauch zurückzuziehen und anschließend ein anderes Instrument zur Durchführung einer bestimmten Aufgabe ganz unproblematisch durch den auskleidenden Stülpschlauch zu derjenigen Stelle, wo etwas getan werden soll, vorzuschieben. Als Beispiel sei ein Ballondilatationsinstrument genannt.

Besonders mühsam, schwierig und zeitraubend war bisher die Koloskopie, also die Exploration des Dickdarms vom Anus her. Der Darm weist Abbiegungen und häufig Engstellen auf. Demzufolge gehören koloskopische Untersuchungen bisher zu den aufwendigen und für den Patienten unangenehmen Untersuchungen und kommen deshalb für eine breite Anwendung, insbesondere im Sinn regelmäßiger Vorsorgeuntersuchungen, kaum in Betracht. Das Umgehen mit einem Koloskop erfordert einen hierin besonders erfahrenen Arzt und besonders erfahrenes Hilfspersonal.

Diee Situation ist besonders nachteilig, weil Anomalien der Darmwand, beispielsweise Polypen, Adenome und Karzinome, in vielen Gegenden der Welt immer zahlreicher werden und weil eine möglichst frühzeitige Erkennung die Heilungsschancen für den betreffenden Patienten ganz erheblich erhöht bzw. zu einer erheblichen Lebensverlängerung führt.

Insofern ist es äußerst wünschenswert, ein Endoskop zur Verfügung zu haben, das unkomplizierter und risikoloser längs des Darms bewegbar ist und das auch von einschlägig nicht so erfahrenen Ärzten bedient werden kann.

Bisher mußte der Patient bei der Durchführung einer Koloskopie wiederholt umgelagert werden und mußte durch gerichtetes Drücken auf den Leib des Patienten versucht werden, die Darmbiegungen so weit zu begradigen, daß das Endoskop einigermaßen gut vorgeschoben werden konnte. Durch die Erfindung wird auf medizinischem Gebiet eine entscheidende Verbesserung gerade bei der Koloskopie erreicht, insbesondere weil durch die Auskleidung des Darms mit dem schützenden, glatt-führenden, äußeren Bereich des Stülpschlauchs die Gefahr der Beschädigung oder gar der Perforation der empfindlichen Darmwand wesentlich reduziert wird. Durch die Erfindung wird die totale Koloskopie bis hin zum Übergang vom Dickdarm in den Dünndarm für eine echte Breitenanwendung, beispielsweise im Sinne allgemeiner Vorsorgeuntersuchungen in sinnvollen Zeitabständen ab einem bestimmten Lebensjahr, zugänglich. Es ergibt sich eine Personalersparnis bei den Untersuchungen.

Die Erfindung und Weiterbildung der Erfindung werden nachfolgend anhand eines teilweise schematisiert dargestellten Ausführungsbeispiels noch näher erläutert. Es zeigt:
- **Fig. 1**: in schematisierter Form eine Vorrichtung zum Bewegen eines Endoskops längs des Darms;
- **Fig. 2**: im Längsschnitt und in größerem Maßstab eine erste Antriebseinrichtung der Vorrichtung von **Fig. 1**;
- **Fig. 3**: teilweise geschnitten eine zweite Antriebseinrichtung und eine Schmiermittel-Einpreßeinrichtung der Vorrichtung von **Fig. 1**;
- **Fig. 4**: in schematisierter Form analog zu **Fig. 1** eine weitere Ausführungsform einer Vorrichtung zum Bewegen eines Endoskops längs des Darms;
- **Fig. 5**: in schematisierter Form analog zu **Fig. 1** eine Variante mit Vorratsstraffungselement;
- **Fig. 6**: schematisiert das Distalende des Endoskops mit einem Relativlagesensor.

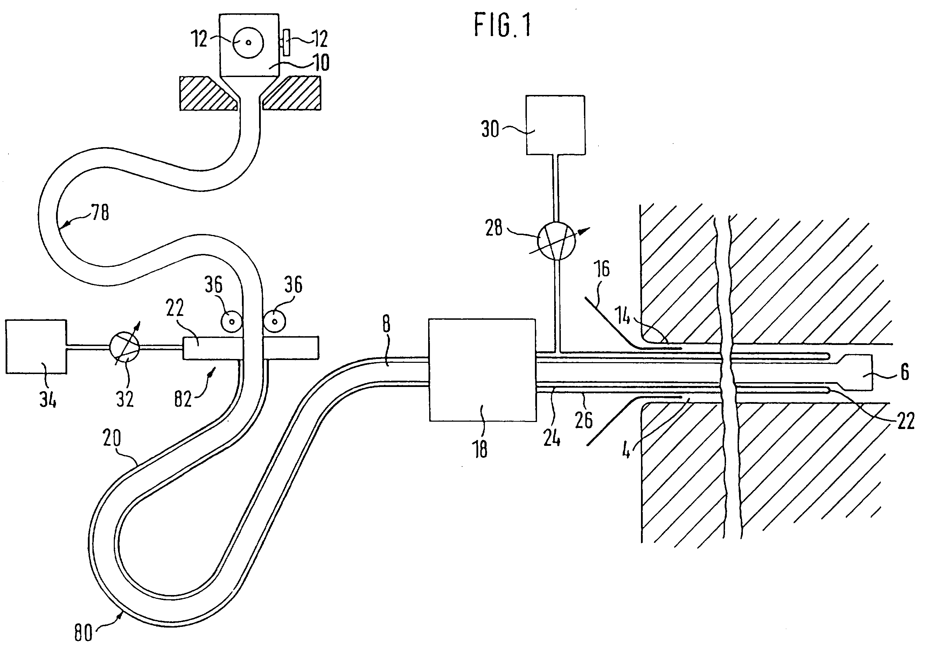

**Fig. 1** zeigt die Vorrichtung in einem Zustand, in der ein Endoskop 2 bereits ein Stück weit in den Darm 4 eines Patienten hineinbewegt worden ist. Das Endoskop 2 besteht im wesentlichen aus einem Distalende 6, einem Endoskopschaft 8 und einem Bedienungsende 10. Der Endoskopschaft 8 ist etwa 3200 mm lang und hat einen Durchmesser von 9 bis 13,5 min. Das Distalende 6 hat einen etwas größeren Durchmesser. An dem Bedienende sind schematisch drehbare Betätigungsscheiben 12 zum Betätigen von Bedienungsdrähten zum Abwinkeln des Distalendes 6 eingezeichnet. In den Anus 14 ist ein Tubus 16 eingeführt, um zu verhindern, daß der Patient den Anus-Schließmuskel schließt.

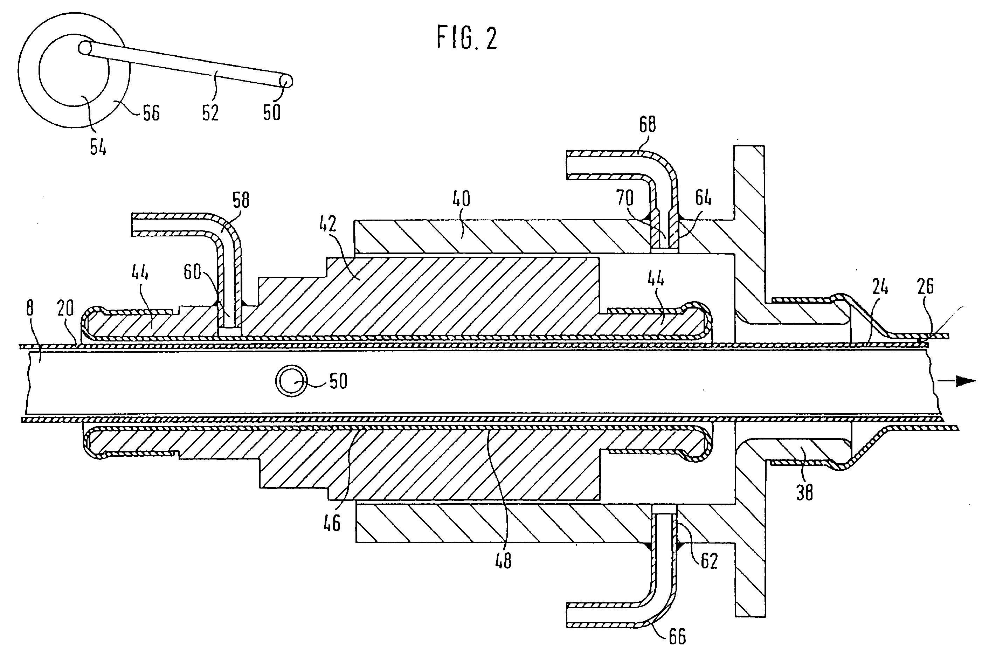

In einem Abstand von etwa 10 bis 20 cm vom Anus 14 befindet sich eine erste Antriebseinrichtung 18, die weiter unten anhand von **Fig. 2** noch genauer beschrieben wird. Ein Stülpschlauch 20 ist mit seinem hinteren Ende an einem plattenartigen Teil 22 befestigt und umgibt von hier aus bis nahe zum vorderen Ende des Endoskops 2 den Endoskopschaft 8. Nahe dem vorderen Ende des Endoskops 2 ist der Stülpschlauch 20 um 180° nach außen und dann wieder zurück umgestülpt und führt von dort wieder zurück zu der ersten Antriebseinrichtung 18. Das vordere Ende des Stülpschlauchs 20 wird Umstülpbereich 22 genannt. Zwischen dem Umstülpbereich 22 und der ersten Antriebseinrichtung weist der Stülpschlauch 20 somit einen inneren Bereich 24 und einen äußeren Bereich 26 auf.

Der Raum zwischen dem inneren Bereich 24 und dem äußeren Bereich 26, rechts in **Fig. 1** abgeschlossen durch den Umstülpbereich 22 und links in **Fig. 1** zeitweise abgeschlossen innerhalb der ersten Antriebseinrichtung 18, kann mittels einer Pumpe 28 mit Flüssigkeitsdruck beaufschlagt werden. Die Pumpe 28 saugt die Flüssigkeit aus einem Vorratsbehälter 30 an. Als Flüssigkeit kann Wasser mit zugesetztem Benetzungsmittel verwendet werden.

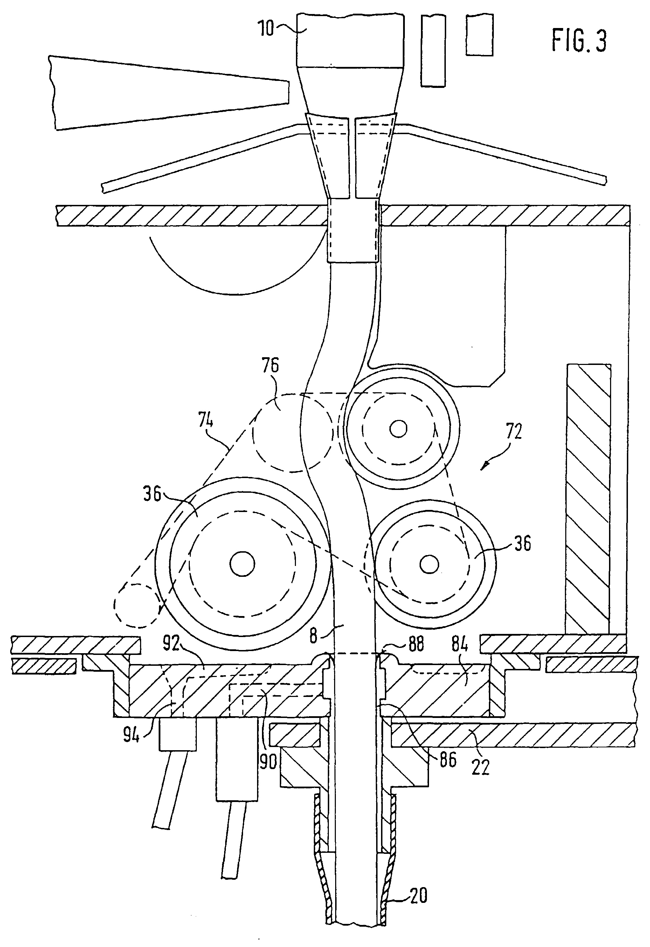

Zwischen der ersten Antriebseinrichtung 18 und der Platte 22 ist der Endoskopschaft 8 mit dem umgebenden Schlauch 20 zu einer ausgebuchteten Form gelegt. Im Bereich der Platte 22 kann Schmiermittel in den Raum zwischen dem Endoskopschaft 8 und dem Schlauch 20 eingepreßt werden, wie weiter unten anhand von **Fig. 3** noch genauer beschrieben wird. In **Fig. 1** ist eine Schmiermittelpumpe 32 und ein zugeordneter Vorratsbehälter 34 für Schmiermittel eingezeichnet. Bei dem Schmiermittel kann es sich zum Beispiel um Pflanzenöl handeln.

Auf der dem Schlauch 20 abgewandten Seite der Platte 22 ist ein Paar von Antriebsrädern 36 vorgesehen, die mit ihrem rinnenförmigen Außenumfang von zwei Seiten her mit dem Endoskopschaft 8 in reibschlüssigem Eingriff sind. Diese Antriebsräder 36 bilden zusammen mit einem nicht eingezeichneten Elektromotor eine zweite Antriebseinrichtung 72.

Im Längenbereich zwischen den Antriebsrädern 36 und dem Bedienungsende 10 des Endoskops 2 ist der Endoskopschaft 8 ebenfalls in eine ausgebuchtete Form gelegt.

In **Fig. 2** erkennt man wiederum den Endoskopschaft 8 und den Stülpschlauch 20. Rechts in **Fig. 2** erkennt man den inneren Bereich 24 und den äußeren Bereich 26 des Stülpschlauchs 20. Das Ende des äußeren Bereichs 26 ist an einem nach rechts weisenden Schlauchstutzen eines Zylinders 40 befestigt.

In dem Zylinder 40 ist eine kolbenartig ausgebildete Hülse 42 angeordnet, die mit ihrem Außenumfang in der Bohrung des Zylinders 40 in Axialrichtung bewegbar geführt ist. Die beiden Axialenden der Hülse 42 sind als Schlauchstutzen 44 ausgebildet. Ein Schlauchstück 46 erstreckt sich längs in einer durchgehenden Innenbohrung 48 der Hülse 42 und ist an seinen beiden Enden nach außen umgestülpt und an den Schlauchstutzen 44 dicht befestigt. Der Endoskopschaft 8 mit dem umgebenden, dort einlagigen Schlauch 20 ist innerhalb des Schlauchstücks 46 durch die Innenbohrung 48 der Hülse 42 hindurchgeführt. Die Wandstärke des Schlauchs 20 beträgt 0,6 bis 1,5 mm. Die Durchmesser der in der Innenbohrung 48 angeordneten Bauteile sind so gewählt, daß zwischen dem Schlauchstück 46 und dem Schlauch 20 sowie zwischen dem Schlauch 20 und dem Endoskopschaft 8 etwas Spiel besteht.

Die Hülse 42 weist ein Paar von diametral vom Außenumfang wegragenden Zapfen 50 auf. Oben links in **Fig. 2** ist angedeutet, wie diese Zapfen 50 über Schubstangen 52 mit einer Exzenterscheibe 54 verbunden sind, die von einem Elektromotor 56 drehend antreibbar ist. Auf diese Weise läßt sich die Hülse 42 in ihrer Axialrichtung hin und her bewegend antreiben. Der Gesamthub dieser Axialbewegung beträgt beim vorliegenden Ausführungsbeispiel 30 mm. Man kann auch mit nur einem Zapfen 50 und einer Schubstange 52 arbeiten.

Anschließend wird die Vorrichtung zunächst mit einer Funktionsweise beschrieben, bei der abwechselnd der Endoskopschaft 8 und der Schlauch 20 als Einheit in einer ersten Richtung bewegt werden und der Endoskopschaft 8 allein in einer entgegengesetzten zweiten Richtung bewegt wird; dies entspricht der in der Beschreibungseinleitung an zweiter Stelle geschilderten Variante der Erfindung. Weiter unten wird dann eine alternative Funktionsweise beschrieben, bei der der vordere Teil des Endoskops 2 mit etwa der halben Geschwindigkeit des inneren Bereichs 24 des Schlauchs 20 bewegt wird; dies entspricht der in der Beschreibungseinleitung an erster Stelle geschilderten Variante der Erfindung.

Der Raum zwischen dem Schlauchstück 46 und der Innenbohrung 48 der Hülse 42 läßt sich über einen Stutzen 58 und durch eine radiale Bohrung 60 der Hülse 42 mit Fluiddruck beaufschlagen. Bei Druckbeaufschlagung dieses Raums bewegt sich das Schlauchstück 46 radial nach innen und preßt den Schlauch 20 auf die Länge der Hülse 42 gegen den Außenumfang des Endoskopschafts 8. Über den Stutzen 58 und die Bohrung 60 laßt sich in dem Raum zwischen dem Schlauchstück 46 und der Innenbohrung 48 wahlweise auch Unterdruck erzeugen, um das Schlauchstück 46 wieder radial nach außen gegen die Innenbohrung 48 zu ziehen und dadurch den Schlauch 20 und den Endoskopschaft 8 wieder freizugeben. Die Druckaufbringung und die Unterdruckaufbringung in dem Raum zwischen dem Schlauchstück 46 und der Innenbohrung 48 sind so gesteuert, daß der genannte Raum im wesentlichen für diejenige Zeitphase unter Druck steht, in der die Hülse 42 von links nach rechts bewegt wird, und für diejenige Zeitphase unter Unterdruck steht, in der die Hülse 42 von rechts nach links bewegt wird. Somit nimmt die Hülse 42 bei ihrer Bewegung von links nach rechts die Einheit aus Endoskopschaft 8 und Schlauch 20 nach rechts mit. Bei der Bewegung der Hülse 42 von rechts nach links wird hingegen die Einheit aus Endoskopschaft 8 und Schlauch 20 nicht mitgenommen. Statt, wie beschrieben, Unterdruck aufzubringen, kann man vereinfachend auch nur den zum Klemmen eingesetzten Überdruck ablassen.

Dort, wo sich der Schlauchstutzen 44 am rechten Ende der Hülse 42 befindet, besteht ein freier Raum zwischen der Hülse 42 und der Innenbohrung des Zylinders 40. In diesen Raum mündet eine untere radiale Bohrung 62 und eine obere radiale Bohrung 64, wobei in der unteren Bohrung 62 ein Stutzen 66 sitzt und in der oberen Bohrung 64 ein Stutzen 68 sitzt. Der Stutzen 68 weist eine Drosselbohrung 70 auf. An den Stutzen 66 ist die in **Fig. 1** gezeichnete Pumpe 28 angeschlossen (wobei dort aus Gründen der leichteren Verständlichkeit die Verbindung zwischen der Pumpe 28 und dem Stülpschlauch 20 rechts von der ersten Antriebseinrichtung 18 gezeichnet worden ist). Durch den Stutzen 66 kann der nicht von der Hülse 42 eingenommene Innenraum des Zylinders 40 mit Fluiddruck beaufschlagt werden. Bei Druckbeaufschlagung wird der Druck in diesem Raum auf etwa 0,7 bar geregelt. Durch den Stutzen 68 kann das Fluid drucklos zurück in den Vorratsbehälter 30 strömen. Aus **Fig. 2** ist deutlich ersichtlich, daß der genannte Innenraum des Zylinders 40 mit dem Raum zwischen dein äußeren Bereich 26 und dem inneren Bereich 24 des Stülpschlauchs 20 in Verbindung steht, so daß dort bei Druckbeaufschlagung ebenfalls der genannte Fluiddruck herrscht.

Der genannte Raum innerhalb des Zylinders 40 und damit auch der Raum zwischen dem äußeren Bereich 26 und dem inneren Bereich 24 des Stülpschlauchs 20 ist jedoch nicht permanent mit Fluiddruck beaufschlagt. Durch entsprechende Regelung der Pumpe 28 oder Regelung des Rückflusses zum Vorratsbehälter 30 wird vielmehr während bestimmter Zeitphasen dafür gesorgt, daß der Druck auf etwa 0,2 bar absinkt. Die Steuerung des zeitlichen Ablaufs ist folgendermaßen:

Wenn sich die Hülse 42 in ihrer Stellung ganz links befindet, wird - wie weiter vorn beschrieben - der Schlauch 20 gegen den Endoskopschaft 8 gepreßt und auf diese Weise auch der genannte Raum im Zylinder 40 nach links hin abgedichtet. Kurz darauf wird der Raum zwischen dem äußeren Bereich 26 und dem inneren Bereich 24 des Stülpschlauchs 20 auf die beschriebene Weise unter Druck gesetzt. Etwa zu der Zeit, zu der die Hülse 42 ihre rechte Endstellung erreicht hat, wird der Druck zwischen dem äußeren Bereich 26 und dem inneren Bereich 24 des Stülpschlauchs 20 reduziert, wie beschrieben, und wird das Anpressen des Schlauchs 20 an den Endoskopschaft 8 aufgehoben, wie beschrieben. Infolgedessen kann dann die Hülse 42 nach links zurückfahren, ohne den Schlauch 20 und den Endoskopschaft 8 mitzunehmen.

**Fig. 3** veranschaulicht die zweite Antriebseinrichtung 72, deren Antriebsräder 36 bereits im Zusammenhang mit **Fig. 1** beschrieben worden sind. Die Antriebsräder 36 stehen über eine Kette 74 mit einem antreibenden Kettenrad 76 in Verbindung, welches seinerseits von einem nicht eingezeichneten Elektromotor in beiden Drehrichtungen antreibbar ist. Während der Zeitphase, in der - wie vorstehend beschrieben - der Schlauch 20 nicht mittels des Schlauchstücks 46 gegen den Endoskopschaft 8 gepreßt wird und die Hülse 42 ihre freie Bewegung von rechts nach links vollführt, zieht die zweite Antriebseinrichtung 72 den Endoskopschaft um 15 mm nach oben, also rückwärts. Der Endoskopschaft 8 kann diese Rückwärtsbewegung ohne Mitnahme des Schlauchs 20 vollführen, weil der Schlauch 20 nirgends mit erheblicher Anpreßkraft gegen den Endoskopschaft 8 gedrückt wird. Durch diese Rückwärtsbewegungen des Endoskopschafts 8 vergrößert sich die Ausbuchtung 78 zwischen der zweiten Antriebseinrichtung 36 und dem Bedienungsende 10 des Endoskops. Während der zuvor beschriebenen Vorwärtsbewegung des Endoskopschafts 8 zusammen mit dem inneren Bereich 24 des Stülpschlauchs 20 verkleinert sich die Ausbuchtung 80 zwischen der ersten Antriebseinrichtung 18 und der Platte 22. Am Ende des Hineinbewegens des Endoskops 2 in den Darm 4 ist die Ausbuchtung 80 praktisch aufgebraucht; es befinden sich etwa 1500 mm Endoskoplänge in dem Darm 4 und etwa 1500 mm Endoskoplänge in der Ausbuchtung 78.

In **Fig. 3** erkennt man ferner weitere Details der Schmiermittel-Einpreßeinrichtung 82 (vergleiche **Fig. 1**). Oberhalb der Platte 22 ist ein plattenartiges Schmiermittel-Zuführbauteil 84 angeordnet. Das Bauteil 84 weist eine Durchgangsöffnung 86 auf, durch die der Endoskopschaft 8 hindurchgeführt ist. In ihrem oberen Bereich ist die Durchgangsöffnung 86 mittels einer Lippendichtung 88 gegenüber dem Endoskopschaft 8 abgedichtet. Im darunter befindlichen Bereich hat die Durchgangsöffnung 86 einen deutlich größeren Innendurchmesser als der Außendurchmesser des Endoskopschafts 8. Dort mündet ein Zuführkanal 90 für das Schmiermittel, der an die Schmiermittelpumpe 32 angeschlossen ist. An seiner Oberseite ist das Bauteil 84 mit einer umlaufenden Vertiefung 92 versehen, von der ein Rücklaufkanal 94 für Schmiermittel, welches zwischen der Dichtung 88 und dem Endoskopschaft 8 möglicherweise nach oben austritt, zurück zu dem Vorratsbehälter 34 führt.

Schmiermittel, welches durch den Kanal 90 unter Druck in den Bereich zwischen der Durchgangsöffnung 86 und dem Endoskopschaft 8 gelangt, breitet sich zwischen dem inneren Bereich 24 des Stülpschlauchs 20 und dem Endoskopschaft 8 auf die gesamte Länge aus. Überschußmengen des Schmiermittels treten im Bereich des Umstülpbereichs 22 des Stülpschlauchs 20 in den Darm 4 aus. Die Schmiermitteleinpreßeinrichtung 82 kann so gesteuert sein, daß jeweils bei Beginn einer Zurückbewegung des Endoskopschafts 8 mittels der Antriebsräder 36 ein kurzer Schmiermittelstoß abgegeben wird.

Mit der zweiten Antriebseinrichtung 72 kann man nicht nur die beschriebenen Rückwärts-Bewegungsschritte von 15 mm durchführen, sondern kann auch den Endoskopschaft 8 wahlweise vorwärts oder rückwärts über eine längere Bewegungsstrecke bewegen. Ein typischer Anwendungsfall für diese Bewegung ergibt sich, wenn das Distalende 6 des Endoskops 2 die innerste Dickdarmbiegung passiert hat. Dann schließt sich ein relativ gerader Dickdarmabschnitt an, den man mittels Ausfahrens des Endoskops ohne Mitnahme des inneren Bereichs 24 des Stülpschlauchs 20 explorieren kann. Es gibt Endoskope, deren Distalende man mit den beschriebenen Drahtzügen um bis zu 160° abwinkeln kann, und bei gegenüber dem Umstülpbereich 22 vorgefahrenem Distalende 6 kann man also quasi auch nach rückwärts blickend den Darm explorieren.

Es wird darauf hingewiesen, daß die weiter vorn beschriebenen Abfolgen der Druckbeaufschlagungen, Druckentlastungen, Unterdruckaufbringungen und Bewegungen des Endoskopschafts 8 mitsamt dem inneren Bereich 24 des Stülpschlauchs 20 und ohne den inneren Bereich 24 des Stülpschlauchs 20 genau umgekehrt ablaufen, wenn man das Endoskop 2 und den Stülpschlauch 20 schrittweise wieder aus dem Darm 4 zurückziehen möchte. Dann kann man den Darm 4 beim Herausbewegen des Endoskops 2 aus dem Darm ein zweites Mal explorieren. Es versteht sich, daß man bei dieser Zurückbewegung mit der zweiten Antriebseinrichtung 72 intermittierend um 15 mm vorschieben statt zurückziehen muß.

Der Stülpschlauch 20 kann aus Silikon bestehen und kann beidseitig z.B. mit Polytetrafluorethylen beschichtet sein.

Der Zylinder 40 kann mitsamt der Hülse 42 um eine Vertikalachse schwenkbar an der Vorrichtung befestigt sein, so daß man die Ausrichtung des patientenseitig aus dem Zylinder 14 austretenden Endoskopschafts 8 relativ zu dem Patienten einstellen kann. Außerdem kann die gesamte Vorrichtung höhenverstellbar sein, damit man die Höhe des aus dem Zylinder 40 austretenden Endoskopschafts 8 an die Höhe, die der Anus des für die Untersuchung gelagerten Patienten hat, anpassen kann. Es versteht sich, daß die Lage des Patienten relativ zu der Vorrichtung im Verlauf der Untersuchung beibehalten werden sollte; insbesondere sollte der Abstand zwischen dem Anus 14 und der ersten Antriebseinrichtung 18 konstant sein.

Die beschriebene Vorrichtung hat für den untersuchenden Arzt ferner den Vorteil, daß er mit einem Bedienende 10 des Endoskops 2 arbeiten kann, welches Bedienende 10 stationär auf der Vorrichtung angebracht ist. Das Hineinbewegen und das Herausbewegen des Endoskopschafts 8 ist ohne Einfluß auf die stationäre Position des Bedienendes 10. Der Arzt kann das Hineinbewegen und das Herausbewegen des Endoskopschafts bequem durch elektrische Schalter an der Vorrichtung steuern. Vor Biegungen des Darms winkelt er das Distalende 6 des Endoskops 2 so ab, daß dieses im wesentlichen in Richtung des vor dem Distalende 6 liegenden Darmverlaufs weist. Alternativ ist es z.B. möglich, den Stülpschlauch 20 und den Endoskopschaft 8 allmählich immer weiter in den Darm 4 hineinzuschieben in einem Zustand, bei dem das vordere Ende des Distalendes 6 geringfügig hinter (also in **Fig. 1** links von) dem Umstülpbereich 22 zurück ist. Dann läuft dem vorderen Ende des Distalendes 6 stets der Umstülpbereich 22 geringfügig voraus, so daß während des Hineinschiebens das Distalende 6 nie in Gefahr ist, mit der Darmwand in Berührung zu kommen.

Aufgrund der vorangegangenen Beschreibung ist klar, daß bei dem beschriebenen Ausführungsbeispiel die zweite Antriebseinrichtung 72 zugleich diejenige Antriebseinrichtung darstellt, mit welcher der Endoskopschaft 8 bedarfsweise ein größeres Stück vorwärts oder rückwärts bewegt werden kann, unabhängig von dem Stülpschlauch 20.

Es wird darauf hingewiesen, daß man beispielsweise statt der insbesondere anhand der **Fig. 2** beschriebenen Klemmeinrichtung auch mit zwei Antriebsrädern analog den Antriebsrädern 36 der zweiten Antriebseinrichtung 72 arbeiten kann. Für das Zurückbewegen des Endoskopschafts 8 relativ zu dem Stülpschlauch 20 kann man beispielsweise den Achsabstand dieser beiden Antriebsräder geringfügig vergrößern und dadurch die Klemmwirkung zwischen dem Stülpschlauch 20 und dem Endoskopschaft 8 aufheben.

Aus der vorstehenden Beschreibung ist ferner ersichtlich, daß streng genommen jeweils der Endoskopschaft 8 mitsamt dem Distalende 6 vorwärts- und rückwärtsbewegt wird, nicht jedoch das Bedienende 10.

Anschließend wird die andere Variante der Bewegungs-Funktionsweise der Vorrichtung beschrieben:

Man kann zu denjenigen Zeitphasen, in denen die erste Antriebseinrichtung 18 eine Bewegung des Stülpschlauchs 20 und des Endoskopschafts 8 in die erste Richtung leistet, z.B. mittels der zweiten Antriebseinrichtung 72 dafür sorgen, daß der Endoskopschaft 8 mit der halben Geschwindigkeit oder um die halbe Strecke in der ersten Richtung bewegt wird wie die Hülse 42. Mit anderen Worten: Der Endoskopschaft vollführt bei Bewegungen in der ersten Richtung eine zurückbleibende Gleitbewegung relativ zu dem Stülpschlauch 20. In diesem Fall hat man streng genommen keine Bewegungsschritte des Endoskopschafts 8 in die zweite Richtung mehr. Die Voraussetzung hierfür, daß nämlich die Reibung zwischen dem Schlauchstück 46 und dem Außenumfang des Stülpschlauchs 20 in der ersten Antriebseinrichtung 18 die Reibung größer ist als zwischen dem Endoskopschaft 8 und dein Stülpschlauch 20, ist gegeben. Wegen der sich allmählich aufbrauchenden Ausbuchtung 80 wird der Endoskopschaft 8 durch die Antriebsräder 36 dort mit der gleichen Geschwindigkeit rückwärts gezogen wie sich der vordere Teil des Endoskopschafts 8 vorwärts in den Darm 4 hineinbewegt.

Man kann sogar so weit gehen, die Bewegung des Endoskopschafts 8 und des Stülpschlauchs 20 in die erste Richtung kontinuierlich statt intermittierend durchzuführen. Wenn man z.B. die insbesondere anhand von **Fig. 2** beschriebene Klemmeinrichtung bzw. erste Antriebseinrichtung durch ein in reibschlüssigem Eingriff mit dem Stülpschlauch 20 stehendes Antriebsräderpaar ersetzt und mit einer weiteren Antriebseinrichtung analog der zweiten Antriebseinrichtung 72 dafür sorgt, daß sich der Stülpschlauch 20 bzw. der innere Bereich 24 des Stülpschlauchs 20 mit der doppelten Geschwindigkeit wie der Endoskopschaft 8 in die erste Richtung bewegt, hat man ebenfalls das Ziel erreicht, den Stülpschlauch 20 und den Endoskopschaft 8 gemeinsam passend im Darin 4 vorwärts zu bewegen.

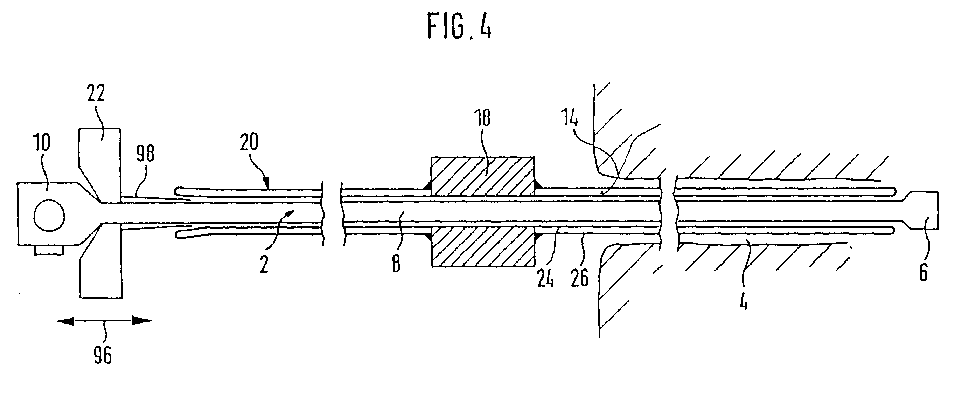

Die Ausführungsform gemäß **Fig. 4** unterscheidet sich von der bisher beschriebenen Ausführungsform gemäß **Fig. 1** bis **3** im wesentlichen dadurch, daß der Stülpschlauch 20 in seinem links von der ersten Antriebseinrichtung 18 befindlichen Bereich nach außen umgestülpt und mit seinem dortigen Ende an der ersten Antriebseinrichtung 18 befestigt ist. Zwischen der ersten Antriebseinrichtung 18 und der Platte 22 hat der Endoskopschaft 8 mitsamt dem Stülpschlauch 20 einen geraden Verlauf statt der Ausbuchtung 80. Das Bedienende 10 des Endoskops 2 sitzt unmittelbar an der Platte 22. Die Platte 22 ist in Längsrichtung des Endoskopschafts 8 bewegbar, s. Pfeil 96. Ein Beispiel hierfür ist die Anbringung der Platte 22 auf einem in Horizontalrichtung verfahrbaren Schlitten, wobei eine nicht eingezeichnete, zweite Antriebseinrichtung vorgesehen ist, um die Platte 22 mindestens in **Fig. 4** nach links gerichtet anzutreiben.

Beim Hineinbewegen des Endoskopschafts 8 in den Darm 4 verkürzt sich derjenige Abschnitt des Stülpschlauchs 20, der sich links von der ersten Antriebseinrichtung 18 befindet, analog dazu, wie sich derjenige Abschnitt des Stülpschlauchs 20, der sich rechts von der ersten Antriebseinrichtung 18 befindet, verlängert. Mit anderen Worten: Der innere Bereich 24 des Stülpschlauchs 20 wird schrittweise durch die erste Antriebseinrichtung 18 hindurchbewegt. Die Bewegungen des Endoskopschafts 8 in die zweite Richtung werden durch angetriebene Bewegungen der Platte 22 nach links in **Fig. 4** erzeugt. Es ist selbstverständlich möglich, auch die Ausführungsform gemäß **Fig. 4** so zu konstruieren, daß die Bewegungen in die erste Richtung und/oder in die zweite Richtung kontinuierlich durchgeführt werden, wie weiter vorne im Zusammenhang mit der ersten Ausführungsform beschrieben.

In **Fig. 4** erkennt man ferner ein ringförmiges, schürzenartiges Teil 98, welches nach rechts weisend an der Platte 22 befestigt ist und in den Raum zwischen dem inneren Bereich 24 des Stülpschlauchs 20 und dem Endoskopschaft 8 ragt, wobei der innere Bereich 24 des Stülpschlauchs 20 möglichst gut abgedichtet mit dem Teil 98 in Kontakt ist. Aus dem Raum innerhalb des schürzenartigen Teils 98 kann Schmiermittel in den Raum zwischen dem inneren Bereich 24 des Stülpschlauchs 20 und der Außenoberfläche des Endoskopschafts 8 eingepreßt werden, analog wie im Zusammenhang mit der ersten Ausführungsform geschildert.

Die Ausführungsform gemäß **Fig. 4** hat gegenüber der ersten Ausführungsform den Vorteil, daß man mit einem handelsüblichen Koloskop mit einer Länge von gut 1500 mm arbeiten kann. Am Anfang befindet sich dieses Endoskoplänge zwischen dem Anus 14 und der Platte 22. Am Ende des Hineinbewegens des Endoskopschafts 8 in den Darm 4 ist die Platte 22 an die erste Antriebseinrichtung 18 herangerückt; die Koloskoplänge befindet sich zwischen der ersten Antriebseinrichtung 18 und dem Explorationsende im Darm 4.

Am Ausführungsbeispiel gemäß **Fig. 4** wird besonders deutlich, daß bei Ausführungsformen der Vorrichtung ohne Vorrat von Schlauch 20 und Endoskopschaft 8 (Vorrat 80) bei der zweiten Variante der Bewegungsfunktionsweise eine rückwärts gerichtete, hemmende Kraft auf den Endoskopschaft 8 ausgeübt werden muß, um die Bewegungsgeschwindigkeit des vorderen Teils des Endoskopschafts 8 auf im wesentlichen die Hälfte der Bewegungsgeschwindigkeit des vorderen Teils des inneren Bereichs 24 des Schlauchs 20 einzustellen. Dies wird bei der Ausführungsform gemäß **Fig. 4** durch eine Bewegung der Platte 22 nach rechts mit entsprechender Geschwindigkeit geleistet. Insofern spricht man bei der zweiten Variante der Bewegungsfunktionsweise sinnvoller von einer Bewegungsbestimmungseinrichtung als von einer zweiten Antriebseinrichtung.

Während bei der ersten Variante der Bewegungsfunktionsweise die Aufbringung einer hohen Anpreßkraft zwischen dem inneren Bereich 24 des Schlauchs 20 und dem Endoskopschaft 8 mittels des Schlauchstücks 48 in der Hülse 42 und auch eine beträchtliche Anpreßkraft zwischen dem inneren Bereich 24 des Schlauchs 20 und dem Endoskopschaft weiter vorn im Darm 4 durchaus vorteilhaft war, um den vorderen Teil des inneren Bereichs 24 des Schlauchs 20 und den vorderen Teil des Endoskopschafts 8 als Einheit zu bewegen, ist es bei der zweiten Variante der Bewegungsfunktionsweise günstiger, wenn diese Anpreßkräfte tendenziell etwas niedriger sind, um die Relativ-Gleitbewegung zwischen dem schnelleren Schlauch 20 und dem langsameren Endoskopschaft 8 nicht übermäßig zu behindern. Andererseits ist es bei der zweiten Variante günstig, für einen recht hohen Reibungskoeffizienten zwischen dem Schlauchstück 46 und dem Außenumfang des inneren Bereichs 24 des Schlauchs 20 zu sorgen, z.B. durch eine schleifpapierartige Innenoberfläche des Schlauchstücks 46.

Versuche haben ergeben, daß es durchaus möglich ist, einerseits genügend Vortriebskraft auf den inneren Bereich 24 des Schlauchs 20 auszuüben und durch den Mitnahmeeffekt zwischen dem inneren Bereich 24 des Schlauchs 20 und dem Endoskopschaft 8 Vortriebskraft auf den Endoskopschaft 8 auszuüben und andererseits mittels der Bewegungsbestimmungseinrichtung 72 eine derartige rückwärtsgerichtete Kraft auf den Endoskopschaft auszuüben, daß er mit dem beschriebenen Geschwindigkeitsverhältnis gegenüber dem inneren Bereich 24 des Schlauchs 20 zurückbleibt.

Der Druck im Raum zwischen dem äußeren Bereich 26 und dem inneren Bereich 24 des Schlauchs 20 beträgt vorzugsweise 0,4 bis 1,2 bar, besonders bevorzugt 0,6 bis 0,8 bar. Der Druck, mit dem das Schlauchstück 46 nach innen gepreßt wird, beträgt vorzugsweise 0,8 bis 1,6 bar, besonders bevorzugt 1 bis 1,3 bar, wobei dieser Druck zur Sicherung der dortigen Abdichtung etwas höher sein sollte als der Druck zwischen dem inneren und dem äußeren Bereich des Schlauchs 20. Der Druck, mit dem Schmiermittel eingepreßt wird, beträgt vorzugsweise 0,2 bis 0,8 bar. Sämtliche, in der Anmeldung genannten Druckwerte bedeuten Überdruck über dem athmosphärischem Druck. Die Wandstärke des Schlauchs 20 beträgt vorzugsweise 0,8 bis 1,2 mm, besonders bevorzugt 0,9 bis 1,1 mm.

Es wird darauf hingewiesen, daß die anhand von **Fig. 2** beschriebene Art des Antriebs der Hülse 42 in Längsrichtung nur eine von mehreren möglichen ist. Eine andere, ebenfalls bevorzugte Möglichkeit ist das Vorsehen einer in Längsrichtung verlaufenden Zahnstange an der Hülse 42, mit der ein von einem Elektromotor angetriebenes Zahnrad kämmt. Dies hat gegenüber dem weiter vorn beschriebenen Kurbeltrieb den Vorteil einer konstanten Geschwindigkeit. Es ist problemlos möglich, die Hülse 42 für einen Bewegungshub von bis zu 25 cm oder sogar noch mehr auszulegen.

Es versteht sich, daß man sinnvollerweise die Relation der Bewegungsgeschwindigkeit der Hülse 42 und die durch die Bewegungsbestimmungseinrichtung 72 definierte Bewegungsgeschwindigkeit des Endoskopschafts 8 durch eine geeignete Steuerung so aufeinander abstimmt, daß sich das beschriebene Geschwindigkeitsverhältnis ergibt. Eine geeignete Steuerung hierfür ist mit dem Wissen des Durchschnittsfachmanns erstellbar.

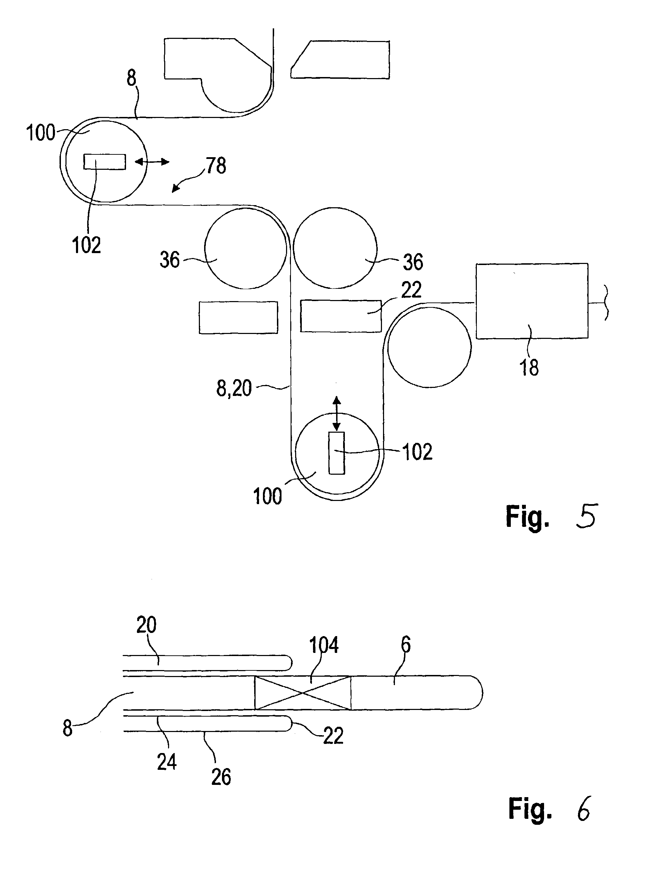

Anhand von **Fig. 5** wird veranschaulicht, daß man für die Ausbuchtung 80 und für die Ausbuchtung 78 jeweils ein Vorratsstraffungselement in Gestalt einer Umlenkrolle 100 vorsehen kann. Jede der Umlenkrollen 100 steht unter der Wirkung einer nicht eingezeichneten Feder derart, daß die Ausbuchtung 80 bzw. 78 straff gehalten wird. Ferner ist für jede der Umlenkrollen 100 ein schematisiert eingezeichneter Wegaufnehmer 102 vorgesehen. Durch Vergleich der Signale der beiden Wegaufnehmer 102 läßt sich auch ermitteln, ob das Distalende 6 des Endoskopschafts 8 sich ordnungsgemäß etwas vor dem Umstülpbereich 22 des Schlauchs 20 befindet. Wenn eine Abweichung von der Soll-Relativlage ermittelt wird, kann korrigierend eingegriffen werden, z.B. durch geringfügige Erhöhung oder Erniedrigung der Drehzahl der Antriebsräder 36.

Anhand von **Fig. 6** wird eine unmittelbarere Ermittlung der Soll-Relativlage des Distalendes 6 des Endoskopschafts 8 relativ zu dem Umstülpbereich 22 des Schlauchs 20 beschrieben. Ein kurzes Stück zurückgesetzt gegenüber dem vorderen Ende des Endoskops ist außenseitig auf diesem ein an sich bekannter Folienschalter 104 befestigt. Wenn der Folienschalter 104 durch den inneren Bereich 24 des Schlauchs 20 druckbeaufschlagt und dadurch geschlossen wird, wird ein Signal abgegeben, welches bedeutet, daß der Endoskopschaft 8 nicht noch weiter gegenüber der Vorwärtsbewegung des Umstülpbereichs 22 zurückbleiben soll. Aufgrund dieses Signals läßt sich z.B. die Bewegungsbestimmungseinrichtung geringfügig in ihrer Geschwindigkeit verstellen in dem Sinne, daß die Bewegungsgeschwindigkeit des Endoskopschafts 8 etwas größer als bisher gemacht wird. Am vorteilhaftesten ist es, einen Doppelschalter mit zwei in Längsrichtung beabstandeten Schaltstellen vorzusehen. Die Soll-Relativlage des Distalendes 6 ist erreicht, wenn die hintere Schaltstelle geschlossen ist und die vordere Schaltstelle geöffnet ist.

## Patentansprüche

1. Endoskopiegerät mit einem Endoskopschaft (8), der zumindest über eine Teillänge von einem Stülpschlauch (20) ummantelt ist, der einen vorderen äußeren Wandbereich (26) hat, der an seinem einen Ende an einer Antriebseinrichtung (18) fixiert und an einem distalen Endabschnitt (6) des Endoskopschafts (8) zu einem inneren Wandbereich (24) umgestülpt ist, **dadurch gekennzeichnet, daß** der innere Wandbereich (24) des Stülpschlauchs (20) an einem zum distalen Endabschnitt (6) des Endoskopschafts (8) gegenüberliegenden hinteren Endabschnitt zu einem hinteren äußeren Wandbereich umgestülpt ist, der zur Antriebseinrichtung (18) zurückgeführt und an dieser fixiert ist.

2. Endoskopiegerät nach Anspruch 1, **gekennzeichnet, durch** eine unmittelbar auf dem Endoskopschaft (8) vor dem hinteren Endabschnitt des Stülpschlauchs (20) fixierte Platte (22).

3. Endoskopiegerät nach Anspruch 1 oder 2, **dadurch gekennzeichnet, daß** die Antriebseinrichtung (18) Antriebsräder hat, die radial gegen einen Abschnitt des inneren Wandbereichs (24) des Stülpschlauchs (20) derart angepreßt sind, daß über den inneren Wandbereich (24) eine Antriebskraft auf den Endoskopschaft (8) für dessen kontinuierliche Bewegung übertragbar ist.

4. Endoskopiegerät nach Anspruch 3, **dadurch gekennzeichnet, daß** die Bewegungsgeschwindigkeit des Endoskopschafts (8) bezüglich der Bewegungsgeschwindigkeit des inneren Wandbereichs (24) mittels der Platte (22) derart regelbar ist, daß die Bewegungsgeschwindigkeit des inneren Wandbereichs (24) im wesentlichen doppelt so groß ist, wie die Bewegungsgeschwindigkeit des Endoskopshafts (8).
